(19) Europäisches Patentamt
European Patent Office
Office européen des brevets

(11) Veröffentlichungsnummer: **0 278 997**
**A1**

# (12) EUROPÄISCHE PATENTANMELDUNG

(21) Anmeldenummer: **87102213.3**

(51) Int. Cl.4: **A61K 31/645**, A61K 9/66

(22) Anmeldetag: **17.02.87**

Ein Antrag gemäss Regel 88 EPÜ auf Berichtigung der Beschreibung, Seite 2, Zeile 26 liegt vor. Über diesen Antrag wird im Laufe des Verfahrens vor der Prüfungsabteilung eine Entscheidung getroffen werden (Richtlinien für die Prüfung im EPA, A-V, 2.2).

(43) Veröffentlichungstag der Anmeldung:
**24.08.88 Patentblatt 88/34**

(84) Benannte Vertragsstaaten:
**AT BE CH DE ES FR GB GR IT LI LU NL SE**

(71) Anmelder: **SIEGFRIED AKTIENGESELLSCHAFT**

**CH-4800 Zofingen(CH)**

(72) Erfinder: **Radivojevich, Franz, Dr.**
**Ackerstrasse 5**
**CH-4800 Zofingen(CH)**
Erfinder: **Dvorsky, Stephan**
**Hauptstrasse 80**
**CH-4102 Binningen(CH)**

(74) Vertreter: **Jaeger, Klaus, Dr. et al**
**Jaeger & Partner Patentanwälte Pippinplatz 4a**
**D-8035 München-Gauting(DE)**

## (54) Azapropazon-Rektalkapseln.

(57) Weichgelatinekapseln, die Azapropazon gelöst in einer Mischung aus Tetrahydrofurfurylalkohol-polyethylenglycolen und einer physiologisch verträglichen wässrigen Base, beispielsweise NaOH enthalten, werden beschrieben. Diese rektal anwendbaren Weichgelatinekapseln erlauben eine ausreichende Resorption des Wirkstoffes durch den Organismus.

EP 0 278 997 A1

Xerox Copy Centre

## Azapropazon-Rektalkapseln

Die Erfindung betrifft eine rektal anwendbare Darreichungsform von Azapropazon sowie ein Verfahren zur Herstellung einer solchen Darreichungsform.

Azapropazon ist der von der WHO empfohlene Name (INN) für 3-Dimethyl-amino-7-methyl-1,2-(n-propylmalonyl)-1,2-dihydro-1,2,4-benzotriazin gemäß Formel (1).

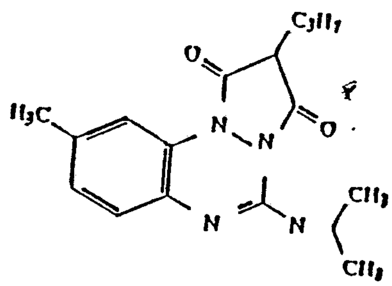

Azapropazon ist ein bekannter Arznei-Wirkstoff mit antiphlogistischer und analgetischer Wirkung, der auch bei allen Formen von Hyperurikämie, insbesondere bei Gicht erfolgreich eingesetzt wird. Herstellung und Eigenschaften der Substanz sind u.a. in DE 14 70 296 C3 beschrieben.

Bei der Überführung von Arzneistoffen in Darreichungsformen werden, wo immer möglich, orale Formen vorgezogen, weil diese besonders bequem zu handhaben sind. Mit Rücksicht auf empfindliche Patienten, welche gegenüber oraler Einnahmen von Arzneimitteln mit Magenbeschwerden reagieren können, sind jedoch mit Vorteil auch bei an sich oral anwendbaren Arzneistoffen Alternativen in Betracht zu ziehen. Als solche stehen neben parenteralen Applikationen, welche aber den Beizug von Arzt oder speziellem Personal bedingen, rektale Anwendungen im Vordergrund. Diese galten bei Azapropazon bis dahin als außer Betracht fallend, weil dieser Wirkstoff infolge seiner geringen Löslichkeit in der Rektumflüssigkeit nicht in ausreichendem Maße resorbiert wird, wenn er in Form herkömmlicher Suppositorien verabreicht wird. Die Menge der im Rektum enthaltenen Flüssigkeit reicht offenbar nicht aus, um die übliche Dosis von 600 bis 700 mg Azapropazon in Lösung zu bringen; jedenfalls haben eingehende kinetische Untersuchungen gezeigt, daß nur eine sehr kleiner Bruchteil des über ein Suppositorium zugeführten Wirkstoffes Azapropazon vom Organismus aufgenommen wird.

Angesichts dieses Standes der Technik liegt der Erfindung die Aufgabe zugrunde, eine rektal anwendbare Darreichungsform von Azapropazon, die gut verträglich ist und genügend absorbiert werden kann, sowie ein Verfahren zur Herstellung einer solchen Darreichungsform zu schaffen.

Diese Aufgabe wird erfindungsgemäß durch die in Anspruch 1 vorgeschlagene Darreichungsform gelöst. Diese Darreichungsform ist in der in Anspruch 14 beschriebenen Weise erhältlich.

Es wird also vorgeschlagen, die rektal anwendbare Darreichungsform von Azapropazon als Weichgelatine-Rektalkapsel auszubilden. Diese ist insofern überraschend, als Weichgelatinekapseln in erster Linie bei oraler Applikation Anwendung finden.

Zwar sind Weichgelatine-Rektalkapseln auch bekannt, jedoch handelt es sich hierbei um eine seltener gewählte Arzneiform, da die gängige Arzneiform bei rektaler Applikation das Suppositorium ist.

Als Lösungsmittel für den Wirkstoff Azapropazon wird erfindungsgemäß ein Gemisch von Tetrahydrofurfurylalkoholpolyethylenglycolen und einer mindestens annähernd äquivalenten Menge einer physiologisch verträglichen wässrigen Base verwendet. Gemische von Tetrahydrofurfurylalkohol-polyethylenglycolen ("THFP") der Formel

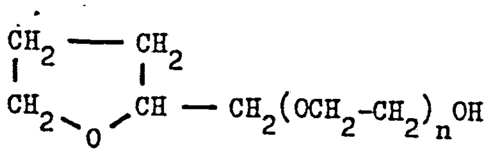

(2)

mit unterschiedlichem Wert von n (vorzugsweise im Bereich von 1 bis 5) sind als Lösungsmittel bekannt und in Verbindung mit Weichgelatinekapseln bereits beschrieben worden (EP-A 143 857). Das mittlere Molekulargewicht der in Azapropazon-Rektalkapseln verwendeten

Tetrahydrofurfurylalkoholpolyethylenglycole soll vorzugsweise im Bereich von 160 bis 240 liegen.

Als zweckmäßig hat sich insbesondere das Handelsprodukt "Tetraglycol" erwiesen, dessen mittleres Molekulargewicht bei 190 liegt. In Betracht kommt beispielsweise auch das Handelsprodukt "Glycofurol 75" mit einem mittleren Molekulargewicht von 168.

Die zweite Komponente des in der Kapselfüllung verwendeten Lösungsmittels ist eine wässrige Lösung einer physiologisch verträglichen Base, die in, bezogen auf den Azapropazon-Gehalt, mindestens annähernd äquivalenter Menge zugegen ist.

Geeignete Basen sind insbesondere Alkalimetall-hydroxide wie Kaliumhydroxid und namentlich Natriumhdyroxid, aber auch organische Basen wie etwa Mono-, Di-und Triethanolamin.

Vorzugsweise enthält die Azapropazon-Rektalkapsel zusätzlich als Lösungsvermittler ein Tensid mit einem HLB-Wert (hydrophilic-lipophilic-balance) im Bereich von 12 bis 16. Wie der empfohlene Wert des Tensids auf der HLB-Skala anzeigt, sind Tenside mit dominierendem hydrophilem Anteil wie beispielsweise das Handelsprodukt Polysorbat 80 besonderes empfehlenswert. Weitere Tenside mit geeignetem HLB-Wert finden sich verzeichnet in H.P. Fiedler, Lexikon der Hilfsstoffe für Pharmazie, 2. Aufl., 1981; als Beispiele seien hier herausgegriffen die Handelsprodukte Tween 21und Tween 80 sowie Cremophor 0, Cremophor El und Cremophor RH 40.

Die mengenmäßige Zusammensetzung der Kapselfüllung mit den oben erwähnten Inhaltstoffen beträgt 20 bis 40 Gew.%, vorzugsweise 28 bis 32 Gew.-% Azapropazon, 3 bis 5 Gew.-% vorzugsweise 3,7 bis 4 Gew.-% Natriumhydroxid, 5 bis 10 Gew.-%, vorzugsweise 7 bis 8 Gew.-% Wasser, 0,1 bis 0,5, vorzugsweise 0,2 bis 0,3 Gew.-%

Polysorbat 80 und Tetrahydrofurfurylalkohol-Polyethylenglycol sowie gegebenenfalls übliche Hilfsstoffe in einer Menge, daß das Gesamtgewicht der Kapselfüllung 100 % beträgt.

Die Kapselhülle besteht üblicherweise im wesentlichen aus Gelatine, Weichmacher, einem üblichen Lichtschutzmittel zum Schutz der Kapselfüllung, Pigmenten und Konservierungsstoffen. Als Weichmacher bzw. Wassergehaltsstabilisatoren können Polyalkohole wie Sorbit, Sorbitan oder Manit verwendet werden. Selbstverstänglich können auch Gemische solcher Polyalkohole zu dem genannten Zweck eingesetzt werden.

Zur Erleichterung der Einführung der Rektalkapsel wird diese auf ihrer Außenseite vorzugsweise mit einem Überzug aus einem physiologisch verträglichen, fett-oder wachsartigen Stoff versehen.

Selbstverständlich sind die Einzelkomponenten der Kapselfüllung, der Kapselhülle und des Überzugs so aufeinander abzustimmen, daß bei Anwendung die Rektalkapsel sich entweder ganz löst oder zumindest soweit erweicht, daß der Wirkstoff Azapropazon freigesetzt werden kann.

Die bevorzugten Dosierungsformen enthalten etwa 600 bis 800 mg Azapropazon in einer Kapsel.

Wie die nachfolgenden Ausführungsbeispiele zeigen, wird Azapropazon, verabreicht in der oben beschriebenen Darreichungsform, im Unterschied zu azapropazonhaltigen Suppositorien gut resorbiert. Dies ist um so überraschender, als das Lösungsmittelgemisch aus Tetrahydrofurfurylalkoholpolyethylenglycol und wässriger Base mit Wasser, und daher auch mit der Rektalflüssigkeit, unbegrenzt mischbar ist, so daß aufgrund der unvermeidlichen Verdünnung der freigesetzten Wirkstofflösung im Rektum ein Ausfallen des Azapropazons zu erwarten wäre. Die Versuche an Suppositotrien hatten nämlich gezeigt, daß ungelöstes bzw. partiell emulgiertes Azapropazon vom Organismus nicht aufgenommen werden kann.

Die Herstellung der Weichgelatine - Rektalkapseln erfolgt in an sich bekannter Weise, indem zuerst die Kapselhüllen vorgeformt und anschließend mit der Kapselfüllung gefüllt werden, oder aus den Materialien für die Kapselfüllung die Kapselhülle in einem Arbeitsgang.

Beispiel 1

1631,06 g Natriumhydroxid werden langsam und portionsweise unter Rühren in 1631,06 g frisch ausgekochtes und auf Raumtemperatur abgekühltes Wasser eingetragen und gelöst.

Parallel dazu werden 104,54 g Polysorbat 80 portionsweise in 24.633,34 g Tetraglycol eingerührt.

In die so hergestellte Mischung wird die parallel hergestellte wässrige Natronlauge portionsweise langsam unter Rühren eingetragen.

In dem so erhaltenen Gemisch werden portionsweise und unter ständigem Rühren 14.000,00 g Azapropazon-Dihydrat dispergiert, wobei die Dispersion in einem verschlossen gehaltenen Behälter unter Stickstoffatmosphäre und Lichtausschluß weitergerührt wird, bis sich alles eingetragene Azapropazon-Dihydrat gelöst hat. Die erhaltene Lösung wird anschließend filtriert und liefert dann 42 kg flüssige Kapselfüllung. Die Füllflüssigkeit wird dann in gebräuchlicher Weise in 60.000 Weichgelatine -Kapselhüllen eingebracht und verkapselt.

Die so hergestellte fertige Darreichungsform enthält 700 mg Azapropazon-Dihydrat pro Weichgelatine-Kapsel.

Die Überlegenheit der Resorption der so hergestellten erfindungsgemäßen Rektalkapseln gegenüber Suppositorien ist aus den Tabellen 1 - 3 ersichtlich.

Tabelle 1: Bioverfügbarkeit der Rektalkapsel

BIOVERFUEGBARKEIT

| Proband | AUC 0 - 72 Rektalkapseln | AUC 0 - 72 i.v. | F |
|---|---|---|---|
| B.A. | 551.07 | 926.46 | 59.5 |
| B.V. | 337.88 | 728.31 | 46.4 |
| H.V. | 633.15 | 1158.02 | 54.7 |
| M.H. | 742.82 | 964.71 | 77.0 |
| W.R. | 534.04 | 1466.56 | 36.4 |
| Z.R. | 857.68 | 1252.36 | 68.5 |
| x | 609.44 | 1082.74 | 57.08 |
| SD | 180.45 | 262.96 | 14.70 |
| VK% | 30 | 24 | 26 |
| geom.x | 585.07 | 1055.72 | 55.43 |

AUC 0-72 in mcg/ml.Std
F in %

Tabelle 2: Plasmaspiegel

Plasmaspiegel ( γ /ml) an Azapropazon (Azapropazon-Äquivalenten) nach 600 mg Azapropazon als Suppositorium

| | 1 h | 2 h | 4 h | 8 h | 24 h |
|---|---|---|---|---|---|
| VP 1 ♂ | 1,2 | 1,6 | 1,5 | 1,5 | ,5 |
| VP 2 ♂ | 2,1 | 2,7 | 2,4 | 2,3 | 3,1 |
| VP 3 ♀ | 1,8 | 2,0 | 2,5 | 2,3 | 3,0 |
| VP 4 ♀ | $< 0,5$ | $< 0,5$ | $< 0,5$ | $< 0,5$ | $< 0,5$ |
| VP 5 ♀ | -- | $< 0,5$ | $< 0,5$ | $< 0,5$ | $< 0,5$ |

Tabelle 3: Ausscheidung

Ausscheidung von Azapropazon in 24 h-Stuhl in Prozent der als Suppositorien verabreichten Dosis

| | % |
|---|---|
| VP 1 ♂ | 99 |
| VP 2 ♂ | 97 |
| VP 3 ♀ | 97 |
| VP 4 ♀ | 94 |
| VP 5 ♀ | 93 |

## Ansprüche

1. Rektal anwendbare Darreichungsform mit einer im wesentlichen aus Gelatine, Glycerin and Wassergehaltsstabilisatoren bestehenden Weichgelatine-Kapselhülle und einer den Wirkstoff enthaltenden flüssigen Kapselfüllung,
dadurch **gekennzeichnet,**
daß die Kapselfüllung zur Hauptsache aus einer Lösung von Azapropazon in einem Gemisch von Tetrahydrofurfurylalkohol-polethylenglycolen besteht und eine (bezogen auf den Azapropazon-Gehalt) höchstens äquivalente Menge einer physiologisch verträglichen wässrigen Base enthält.

2. Darreichungsform nach Anspruch 1,
dadurch **gekennzeichnet,**
daß das Tetrahydrofurfurylalkohol-polyethylenglycol-Gemisch ein mittleres Molekulargewicht von 160 bis 240 aufweist.

3. Darreichungsform nach einem der Ansprüche 1 oder 2,
dadurch **gekennzeichnet,**
daß die Kapselfüllung zusätzlich ein Tensid mit einem HLB-(hydrophilic-lipophilic-balance)-Wert im Bereich von 12 bis 16 enthält.

4. Darreichungsform nach einem der Ansprüche 1 bis 3,
dadurch **gekennzeichnet,**
daß die Kapselfüllung als Base wässrige NaOH-Lösung enthält.

5. Darreichungsform nach einem der Ansprüche 1 bis 4,
dadurch **gekennzeichnet,**
daß die Kapselfüllung, bezogen auf das Flüssigkeitsgewicht, 28 bis 32 Gew.-% Azapropazon, 3,7 bis 4 Gew.-% Natriumhydroxid, 7 bis 8 Gew.-% Wasser und 0,2 bis 0,3 Gew.-% Polysorbat 80 enthällt.

6. Verfahren zur Herstellung einer rektal anwendbaren Azapropazon enthaltenden Weichgelatinekapsel nach einem der Ansprüche 1 bis 5
dadurch **gekennzeichnet,**
daß man zur Herstellung der Kapselfüllung der Weichgelatinekapsel eine Lösung von Azapropazon in einem Gemisch von Tetrahydrofurfurylalkoholpolyethylenglycolen und einer (bezogen auf den Azapropazon-Gehalt) höchstens äquivalenten Menge einer physiologisch verträglichen wässrigen Base benutzt, wobei der Lösung gegebenenfalls übliche Zusatzstoffe zugesetzt sein können.

Europäisches Patentamt

# EUROPÄISCHER RECHERCHENBERICHT

Nummer der Anmeldung

EP 87 10 2213

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 154 523 (RAINSFORD et al.) * Seite 7, Zeilen 17-20 * --- | | A 61 K 31/645<br>A 61 K 9/66 |
| D,A | EP-A-0 143 857 (SIEGFRIED AG) * Seite 5, Zeile 22 - Seite 7, Zeile 19 * --- | | |
| A | CHEMICAL ABSTRACTS, Band 95, Nr. 10, November 1981, Seite 418, Zusammenfassung Nr. 175683p, Columbus, Ohio, US; F. MOOLENAAR et al.: "Manipulation of rectal absorption rate of phenytoin in man", & PHARM. WEEKBL. SCI. ED. 1981, 3(4), 175-80 --- | | RECHERCHIERTE SACHGEBIETE (Int. Cl.4) |
| A | CHEMICAL ABSTRACTS, Band 96, Nr. 6, Februar 1982, Seite 379, Zusammenfassung Nr. 40918u, Columbus, Ohio, US; & JP-A-81 131 514 (NISSAN CHEMICAL INDUSTRIES, LTD, IWAKI SEIYAKU CO., LTD) 15-10-1981 ----- | | A 61 K |

Der vorliegende Recherchenbericht wurde für alle Patentansprüche erstellt.

| Recherchenort | Abschlußdatum der Recherche | Prüfer |
|---|---|---|
| DEN HAAG | 23-10-1987 | BENZ K.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

JAEGER & PARTNER
PATENTANWÄLTE

NEU – NEW
8035 MÜNCHEN-GAUTING
PIPPINPLATZ 4a

PATENTANWÄLTE JAEGER & PARTNER
BERGSTRASSE 48 1/2 · 8035 MÜNCHEN-GAUTING

An das
Europäische Patentamt

8000 München 2

EPA-EPO-OEB
DG 1
27 03 1987

ZUGELASSEN BEIM EUROPÄISCHEN PATENTAMT
ADMITTED TO THE EUROPEAN PATENT OFFICE
AGRÉÉS AUPRÈS DE L'OFFICE EUROPÉEN DES BREVETS

BÜRO MÜNCHEN:
DIPL.-CHEM. DR. KLAUS JAEGER

BÜRO KLEINOSTHEIM:
DR.-ING. HANS H. PONTANI

TELEPHON : (089) 8506091
TELEX : 521777 isar d
TELEFAX : (089) 8503633

IHR ZEICHEN
YOUR REF. :

UNSER ZEICHEN
OUR REF. : SGF-105-EP

DATUM
DATE : 17. März 1987
sr

BETR.
RE. 87 102 213.3
Siegfried AG, Zofingen

Im Anschluß an den Antrag auf Erteilung eines europäischen Patents vom 17. Februar 1987:

Es wird gebeten, in den geltenden Unterlagen auf Seite 2 der Beschreibung, 2. Absatz, Zeile 19, "... eingehende kinetische .." zu ersetzen durch "... eingehende pharmakokinetische ...". ~~Außerdem wird gebeten, die Tabelle 1 auf Seite 7 der Beschreibung durch die anliegend übersandte Tabelle "Bioverfügbarkeit" zu ersetzen.~~

~~Gründe: In der Tabelle 1 wird als Überschrift für Spalte 2 der Ausdruck "Rektokaps" verwendet. Da festgestellt wurde, daß "Rectokaps" eine IR-Markenregistrierung ist, soll diese Überschrift der im übrigen Text gebräuchlichen Bezeichnung "Rektalkapseln" angepaßt werden.~~

~~Die beantragten Änderungen dienen somit der Klarstellung~~.

Jaeger & Partner
Patentanwälte
durch:

(Dr. K. Jaeger)